# EUROPEAN PATENT SPECIFICATION

(11) **EP 2 890 665 B1**
(45) Date of publication and mention of the grant of the patent: **22.05.2019**
(21) Application number: 12883727.5
(22) Date of filing: 31.12.2012
(51) Int. Cl.: C07C 13/70, C07C 2/02, C07C 2/76

(54) **FORMATION OF [2,2]PARACYCLOPHANE AND RELATED COMPOUNDS AND METHODS FOR THE FORMATION OF POLYMERS FROM CYCLOPHANES**
BILDUNG VON [2,2]PARACYCLOPHAN UND VERWANDTEN VERBINDUNGEN SOWIE VERFAHREN ZUR HERSTELLUNG VON POLYMEREN AUS CYCLOPHANEN
FORMATION DE [2,2]PARACYCLOPHANE ET DE COMPOSÉS APPARENTÉS ET PROCÉDÉS POUR LA FORMATION DE POLYMÈRES À PARTIR DE CYCLOPHANES

(30) Priority: 30.08.2012 US 201213599996
(43) Date of publication of application: 08.07.2015
(73) Proprietor: Carver Scientific, Inc., Baton Rouge, LA 70820 (US)
(72) Inventor: CARVER, David, Baton Rouge, LA 70820 (US); REYNOLDS, Sean, Baton Rouge, LA 70820 (US)
(74) Representative: Bond, Christopher William
(86) International application number: PCT/US2012/072335
(87) International publication number: WO 2014/035456

(56) References cited:
- US-A- 3 149 175
- US-A- 3 271 471
- US-A- 5 324 702
- US-A- 6 107 184
- BATTIN-LECLERC ET AL.: 'Experimental and modeling study of the oxidation of xylenes.' INTERNATIONAL JOUMAL OF CHEMICAL KINETICS vol. 38, no. 4, April 2006, pages 284 - 302, XP055249870 Retrieved from the Internet: <URL:http://www.researchgate.net/publicatio n/2175361_Experimental_and_modeling_study_o f_the_oxidation_of_xylenes>

## Description

### FIELD OF THE INVENTION

The present disclosure relates to the field of synthesis of cyclophanes and their method of application and utility as polymer precursors.

### BACKGROUND OF THE INVENTION

Cyclophanes are a subset of organic structures that are well known and characterized. Several excellent reviews and books have been published that cover the methods and applications very well.

Briefly, cyclophanes and other related benzocycloid compounds are organic molecules that have structures where a cyclic carbon or heteroatom substituted chain is attached to two or more positions of an aromatic ring. The term cyclophanes is used to describe compounds that have a relationship or a structure that broadly fits into this structural category.

One of the more highly researched cyclophane compounds is the paracyclophane structure. In one set of cyclophane compounds (as shown in the structures of [n]metacyclophanes (I), [n]paracyclophanes (II) and [n,n']cyclophanes below), we see structure III as a general structure for paracyclophanes.

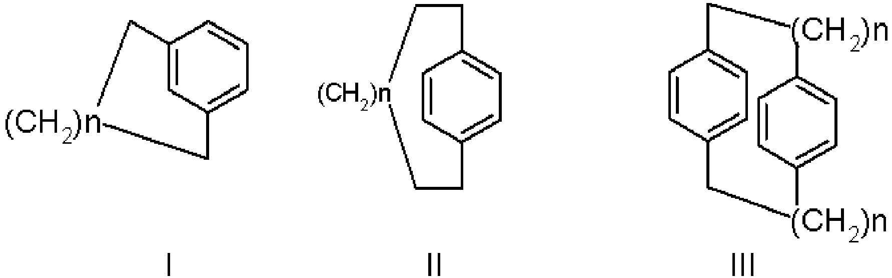

In this substitution pattern we note that the simplest member of the series is where n=1. In this case the molecule is named [2,2']paracyclophane. This molecule and derivatives thereof are an important class of compounds that are able to form a variety of polymer structures. For this reason they are highly desirable organic intermediates that have been used as precursors for conformal coatings for numerous applications. In these applications the molecule shown in III (n=1) is normally heated in a vacuum to produce a significant vapor pressure and to force a disassociation of the molecule into a highly reactive intermediate. This pyrolytic cleavage of the [2.2']paracyclophane results in two molecules of the reactive intermediate p-xylylene (shown below).

Pyrolysis, 600 to 700 °C, 13 to 27 Pa (0.1 to 0.2 torr)

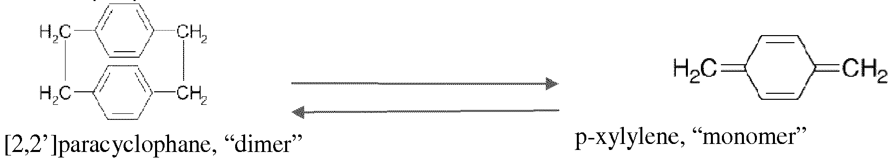

Additionally, the reactive intermediate p-xylylene may be formed from the "dimer" by utilization of a pyrolysis discharge under reduced atmospheric pressure, (ref. Gorham US patent number 3,342,754). This procedure has commonly been called the "Gorham process".

As the structure indicated, the reactive intermediate, p-xylylene is a long-lived intermediate species that has the ability to react to form a highly desirable polymer. In particular this polymer is a conformal coating that has the ability to coat surfaces in relatively uniform layers that are highly resistant to chemical solvents, gases, and biological attack. The p-xylylene is deposited in a vacuum onto a target surface for conformal coating. On the surface it reforms into a repeating unit of poly(p-xylylene), also known as parylene. In the case of no additional substituents on the aromatic rings or the aliphatic side-chains other than hydrogen, this polymer compound is called parylene-N.

### Vacuum deposition (13 Pa (0.1 torr))

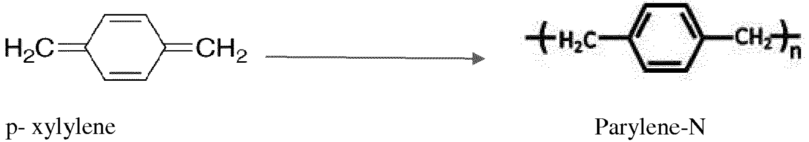

Para-xylylene, as a valuable reactive intermediate, has had its synthesis primarily through the pyrolysis of [2,2']paracylophane. Thus, the synthesis of [2,2']paracyclophane is a critical stable intermediate in the utilization of p-xylylene and the polymer parylene.

Synthesis of [2,2']paracylophane is through the route of the 1,6-Hofmann elimination of quaternary ammonium salts.

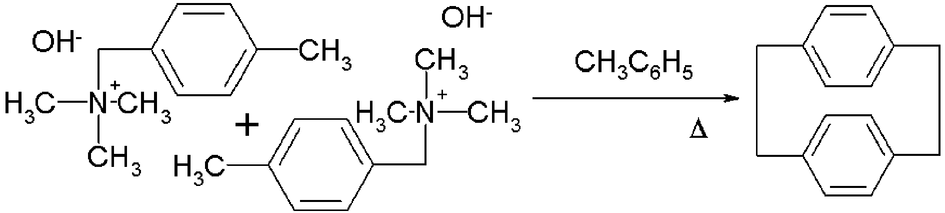

This route going through a quaternary ammonium salt, although widely used, suffers from several drawbacks. The paracylophane is usually produced in low yields using a multi-step processes.

Additionally, due to the low yield and large amount of side-products, extensive purification of the resultant dimer is an additional process procedure.

US 3271417 A discloses 1,2-thiazetidine-3-one-1-oxides and their preparation.

US 6107184 A discloses a method and apparatus for forming thin copolymer layers having low dielectric constants on semiconductor substrates.

US 5324702 A discloses catalytic oxidation and oxidative dehydrogenation using metal-compound-loaded, deboronated hams-lb crystalline borosilicate molecular sieve compositions.

### SUMMARY OF THE INVENTION

The present invention is as defined in the claims. In particular, the present invention is as defined in the following clauses:
1. A method for producing dimers, trimers, oligomers, and polymers from the oxidation of a hydrogen atom alpha to an aromatic ring comprising:
   introducing an organic starting material containing at least one hydrogen atom alpha to an aromatic ring into a flowing stream of gas,
   mixing said organic starting material in the stream of gas with nitrous oxide;
   reacting said hydrogen atom alpha with the aromatic ring to form a reaction product; and
   heating said flowing stream of gas with the reaction product to a reaction temperature to allow oxidation of the hydrogen atom alpha and subsequent formation of an output compound from the group consisting of monomers, dimers, trimers, oligomers, and polymers, according to said reaction temperature.
2. A method as in clause 1 wherein the organic starting material comprises xylene.
3. A method as in clause 1 wherein there are two hydrogen atoms alpha to the same aromatic ring.
4. A method as in clause 1 wherein the aromatic ring is benzene.
5. A method as in clause 1 wherein the output compound is mixed with a cool inert gas.
6. A method as in clause 1 wherein the pressure of the flowing stream of gas with the reaction product being heated to a reaction temperature is increased by applying a back pressure device.
7. A method as in clause 1 further comprising a step of mixing a plasma gas with said flowing stream of gas and the organic starting material and nitrous oxide, forming a reaction mixture.
8. A method as in clause 1 further comprising a step of exposing the output compound to a magnetic field during condensation.
9. A method as in clause 1 further comprising a step of exposing the output compound to an electric field during condensation.

Heretofore, the restriction of the known vacuum (Gorham) process for both the pyrolysis of the dimer and the deposition of the monomer to produce the polymer has increased the cost and limited the utility of the polymer's applications. A method whereby the need for reduced pressure during the process and during the application of the materials is highly desired.

A variety of substitutions for the various hydrogen atoms and substitution of heteroatoms in place of the various carbon atoms in the rings and chains have been made. It would be desirable to have an efficient and effective method for the formation of these compounds, both known and unknown.

Thus, there is a need for an improved synthesis of the stable intermediate dimer of xylylene ([2,2']paracyclophane) and derivatives related to that compound and general structure. Also, a general method for the formation of cyclophanes, and related compounds with various substituents, via a low cost method is desired. Also needed is an improved method to apply the xylylene (or substituted xylylene) monomers to make coatings and other polymer products derived from this reactive intermediate. Even more desirable would be a method to modify the physical properties of the resulting polymers for additional applications and extensions of methods. In the discussion that follows, the example of p-xylylene will be used to illustrate the methods and processes that are taught. The method and processes can be extended to other similar molecules by those versed in the fields of this disclosure, and those molecules will therefore not be explicitly discussed exhaustively.

It is therefore the object of this disclosure to alleviate the costs and problems described in the processes described above. First, a general method for the formation of the example reactive intermediate p-xylylene is shown. Secondly, a method whereby the formation of the stable intermediate chemical compound such as [2,2']paracyclophane ("the dimer"), and related structures, is shown for utility in existing manufacturing processes. Third, it is also shown a direct and economical method for the application of the monomer to the target without the need for reduced pressure, or any pressure change for that matter. Fourth, there will taught a method whereby the physical properties of the resulting products of the reactions may be modified and controlled for specific purposes for the improvement of other processes.

Since the products derived from the methods that follow can be highly controlled for both purity and control of the physical properties, the trademark Puralene™ has been used for products produced by the methods disclosed herein. Substantial deviation under operator control from the normal parylene production process enables Puralene™ to display unique and novel properties not normally associated with traditional parylene products.

In a preferred aspect of the present disclosure an apparatus, which does not form part of the claimed invention, and method to form the example reactive intermediate p-xylylene through the use of a heated a pyrolysis reaction tube into which a flowing stream of a mixture of inert gas and nitrous oxide with xylene vapor in an inert carrier gas at atmospheric pressure with the exit gases nonvolatile reaction products being condensed onto a cooled vessel is disclosed.

In another aspect of the present disclosure a method whereby a chemically reactive intermediate is formed from nitrous oxide allows for the selective formation of p-xylylene in the gas phase at atmospheric pressures.

In another aspect of the present disclosure, an apparatus, which does not form part of the claimed invention, and method to mix cool nonreactive gases into the hot reaction stream, resulting in cooling of the elevated temperature of the reaction gases and thus improving the ability of the reactive intermediates to condense and adhere to the surfaces, is disclosed.

In another aspect of the present disclosure, an apparatus, which does not form part of the claimed invention, and method to have the reaction proceed at an increased pressure and an expansion value at the exit of the heated pyrolysis reaction tube to provide expansion cooling of the hot gases below their inversion temperatures by the Joule-Thomson effect is disclosed.

In another aspect of the present disclosure, an apparatus, which does not form part of the claimed invention, and method using organic starting materials with substituents including chloro, dichloro, methoxy, and methyl are disclosed.

In another aspect of the present disclosure, an apparatus, which does not form part of the claimed invention, and method for using organic starting materials with meta and/or ortho orientation of the substituents on the aromatic rings is disclosed.

In another aspect of the present disclosure, an apparatus, which does not form part of the claimed invention, and methods for the indirect radical formation and/or ionization of the starting material through the reaction of the starting material (e.g. p-xylene) with a plasma and/or combination heating source is disclosed.

In another aspect of the present disclosure, substitution of nitrogen for argon and/or other essentially inert gases is disclosed.

An additional modification of the methods taught above is introduced whereby the physical properties of the resultant polymer or other reaction products can be modified by the use of magnetic and/or electrical fields to provide for controlled physical properties.

### BRIEF DESCRIPTION OF THE DRAWINGS

The foregoing and other aspects, objects, features and advantages of the present disclosure will become better understood with reference to the following description, appended claims, and accompanying drawings, where:
Figure 1 is a schematic drawing of the basic apparatus, which does not form part of the claimed invention, for producing reaction 1;
Figure 2 is a schematic drawing of the basic apparatus, which does not form part of the claimed invention, for producing reaction 2; and
Figure 3 is a high level flowchart that illustrates an exemplary method of producing an augmented permittivity material according to principles of the present disclosure.

### DETAILED DESCRIPTION

### Apparatus (which Apparatus does not form part of the claimed invention) Description For Reaction 1:

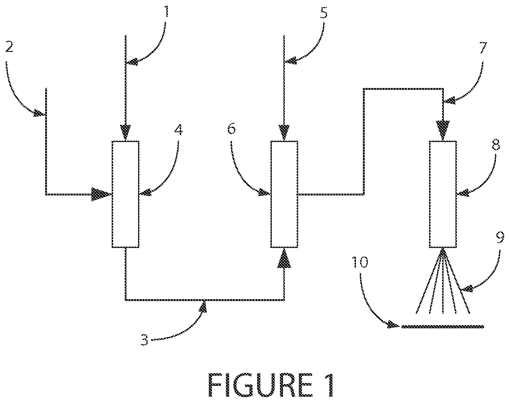

Now referencing Figure 1 where starting material feed 1 is introduced into chamber 4 by utilization of a pumping mechanism (not shown) for liquid or solid feeds. Typically chamber 4 would be a heated tube or other evaporation device to volatilize starting material feed 1. Starting material feed 1 is evaporated and mixed with inert gas 2 in chamber 4. Inert gas 2 may be any of a group of inert gases such as but not limited to Argon. The resulting volatile mixture 3 is transported to chamber 6 and subsequently mixed with nitrous oxide 5, to produce chemical reaction mixture 7. Reaction chamber 8 is typically heated to approximately 450° C to 800° C to enable the reaction and allow the vaporization of the reaction products to be expelled as products 9, onto collection surface 10.

### Apparatus (which Apparatus does not form part of the claimed invention) Description For Reaction 2:

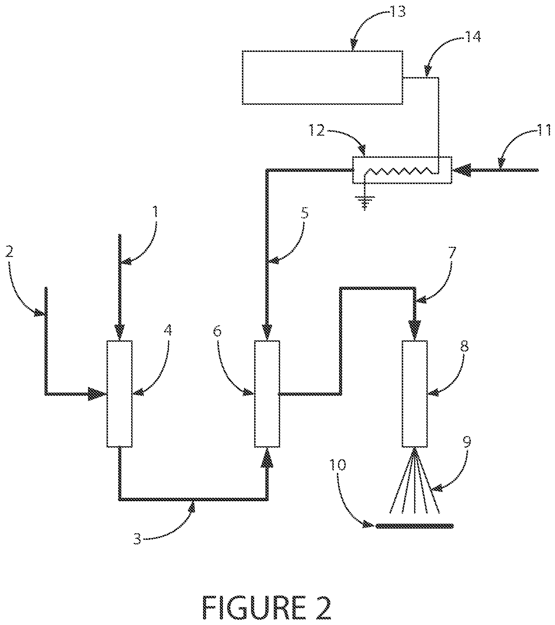

Now referencing Figure 2 where starting material feed 1 is introduced into chamber 4 by utilization of a pumping mechanism (not shown) for liquid or solid feeds. Typically chamber 4 would be a heated tube or other evaporation device to volatilize starting material feed 1. Starting material feed 1 is evaporated and mixed with inert gas 2 in chamber 4. Inert gas 2 may be any of a group of inert gases such as but not limited to Argon. The resulting volatile mixture 3 is transported to chamber 6. The formation of the gaseous plasma 5, is from chamber 12, that is electrically connected via conductor 14 to electrical plasma generator 13. Gas feed 11 is a feed of a suitable gas for conversion to gaseous plasma 5 by induction into chamber 12 resulting in gaseous plasma 5. Volatile mixture 3 is subsequently mixed with gaseous plasma 5 in chamber 6 to produce chemical reaction mixture 7 which is transported to reaction chamber 8. Reaction chamber 8 is typically heated to approximately 450° C to 800° C to enable the reaction and to allow the vaporization of the reaction products to be expelled as products 9, onto collection surface 10.

### Apparatus (which Apparatus does not form part of the claimed invention) Description For Reaction 3:

Now referencing Figure 1 where starting material feed 1 is introduced into chamber 4 by utilization of a pumping mechanism (not shown) for liquid or solid feeds. Typically chamber 4 would be a heated tube or other evaporation device to volatilize starting material feed 1. Starting material feed 1 is heated and in chamber 4. The resulting volatile mixture 3 is transported to chamber 6 and subsequently mixed with nitrous oxide 5, to produce chemical reaction mixture 7. Reaction chamber 8 is typically heated to approximately 450° C to 800° C to enable the reaction and allow the vaporization of the reaction products to be expelled as products 9, onto collection surface 10.

### Apparatus (which Apparatus does not form part of the claimed invention) Description For Reaction 4:

Now referencing Figure 2 where starting material feed 1 is introduced into chamber 4 by utilization of a pumping mechanism (not shown) for liquid or solid feeds. Typically chamber 4 would be a heated tube or other evaporation device to volatilize starting material feed 1. Starting material feed 1 is evaporated in chamber 4. The resulting volatile mixture 3 is transported to chamber 6. The formation of the gaseous plasma 5, is from chamber 12, that is electrically connected via conductor 14 to electrical plasma generator 13. Gas feed 11 is a feed of a suitable gas for conversion to gaseous plasma 5 by induction into chamber 12 resulting in gaseous plasma 5. Volatile mixture 3 is subsequently mixed with gaseous plasma 5 in chamber 6 to produce chemical reaction mixture 7 which is transported to reaction chamber 8. Reaction chamber 8 is typically heated to approximately 450° C to 800° C to enable the reaction and to allow the vaporization of the reaction products to be expelled as products 9, onto collection surface 10.

### Reaction 1:

To form the reactive intermediate p-xylylene, a pyrolysis reaction tube was constructed. The main element in the heated area was an Inconel (nickel alloy 600) tube (0.826 cm (0.325") OD X 0.704 cm (0.277") ID X 152 cm (60") length, Grainger # 3ACP8). The tube was electrically heated to the indicated temperatures. A flowing stream of argon gas mixture comprised of nitrous oxide (Airgas # UM1070) with xylene vapor (Aldrich #134449-4L) in the carrier gas of argon (Airgas#UM1006) was introduced to the tube at a total flow rate of 20 to 100 mL/minute at a temperature of 450°C to 630°C and at atmospheric pressure. The ratio of gases is adjusted to provide approximately molar stoichiometric ratios of 1:1 (xylene to nitrous oxide). The exit gas was comprised of a clear colorless flow of reactive gas. Condensation of the gas onto a cooled glass vessel resulted in the deposition of a colorless to cream colored solid. This solid is partially soluble in 95% ethanol. The solid was compared to a sample of [2,2']paracyclophane (Aldrich #P225-5G-A) by GC analysis (SRI#310, 15m, megabore column, FID detector) and was shown to give identical retention times. At higher temperatures (650° C to 800°C) the output of the reaction tube is sufficiently hot enough to maintain the monomeric p-xylylene in monomeric form. Rapidly cooling of the monomer onto a surface results in a liquid condensation of the monomer and rapid polymerization of the monomer into a polymer. Comparison of the film thus produced appears to be identical to parylene film produced by the Gorham process. (Permittivities both measured to be 3, electric breakdown strengths are identical at 100 V/micron, and solubility in both hot and cold solvents are below detectable levels.)

In this reaction it is presumed, but not proven, that the reactive p-xylylene reactive intermediate is formed and subsequently dimerized in the reaction tube or during condensation onto the glass container. This reaction used to synthesize the dimer, in comparison with the known "Gorham process", results in a vast improvement in the overall synthesis yield of the dimer and also results in a vast improvement in the purity of the dimer directly from the reaction. It is understood that variation in the stoichiometric amounts of the reactants may be adjusted to provide for greater or lesser yield with associated purities varying to provide a more economical process or better overall production efficiency without substantially deviating from the scope of this invention. Subsequent purifications of the materials from this reaction can be performed on this material in a manner that is much easier to accomplish than with previously taught processes.

The reaction is shown below.

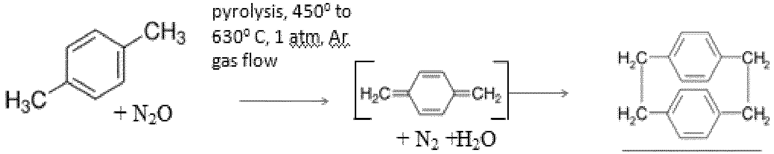

As the reaction temperature is increased to >650°C, the deposition of the xylylene monomer can proceed directly onto a solid substrate target without the necessity for isolation of the intermediate dimer. Deposition of the exit gas at above 650°C reaction temperature upon a cool glass plate resulted in formation of an ethanol insoluble substance that displays characteristics of a parylene polymer. However, solubility characteristics clearly show that the material is insoluble in all common solvents (i.e. hexane, xylene, ethyl acetate, ethanol, water).

Increased amounts of nitrous oxide result in partial and/or complete oxidation of xylene with reduced formation of the desired cyclophane or its polymer. Close control of the stoichiometry is desired in this gas phase reaction.

It is hypothesized that the reaction mechanism proceeds through a route involving the prior decomposition of nitrous oxide. Nitrous oxide is energetically unstable molecule that can be thermally decomposed at elevated temperatures. Products of the reaction are diatomic nitrogen and monoatomic oxygen. The monoatomic oxygen is able to react with itself to form diatomic oxygen, but this reaction is relatively slow. Estimates vary determining the temperature that pure thermal decomposition occurs, but estimates of 1100° C are often cited. Catalysis of this reaction as shown below in equation 1 is known to occur with a variety of metal oxides and mixed metal oxides. Some temperatures used for nitrous oxide decomposition with certain catalysts are as low as 350° C.

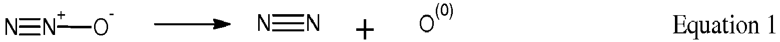

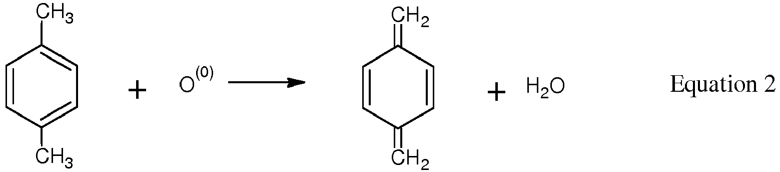

The reactive species for the process is very likely the monoatomic oxygen produced from the decomposition of the nitrous oxide. In this sense, the nitrous oxide can be viewed as a convenient carrier for the delivery of the reactive intermediate monoatomic oxygen. One would then think that diatomic oxygen could be used to perform the same reaction. However, use of diatomic oxygen under the same conditions as the nitrous oxide fails to produce the reaction. With activation of the reaction with a hot plasma spark, ignition of the xylene takes place with highly colored by-products as well as substantially decreased yields of the desired product.

In a similar manner to the nitrous oxide reaction, pure diatomic oxygen can be utilized as a reactant. However, to produce substantial yields of the desired products, activation of the oxygen is necessary. It is presumed but not proven that the activation of the oxygen is due to the excitation of the oxygen molecule to produce monoatomic oxygen as shown in Equation 3.

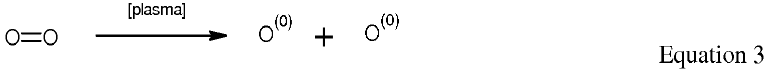

The reaction with monoatomic oxygen produced in this manner in Equation 2 thus proceeds in a manner similar to that of the nitrous oxide decomposition route.

The chemical reaction mechanistic explanations offered in this disclosure are for a proposed framework for discussion of results, and in no way limit to effect the actual results of the procedures of the invention disclosed herein. Alternative explanations may fully describe some results contained herein and may be suitable for intellectual discussion, but they do not in any way detract from the invention described in this disclosure or the analysis described.

Cooling of the elevated temperature gases exiting from the reaction tube is necessary. If the reaction gas is at too high of a temperature, the ability of the reactive intermediate to condense and adhere to a surface is greatly reduced. To this end, a device to mix cool nonreactive gases into the hot reaction stream has been devised. Additionally, the reaction may proceed at increased pressure (above atmospheric pressure), and an expansion valve may be used at the exit of the reaction to provide Joule-Thomson effect cooling of the hot gas when the gas is below its inversion temperature. Methods to do both are well known, and may be optionally incorporated into the procedures.

The method may be extended to other substrates such as the ones shown below.

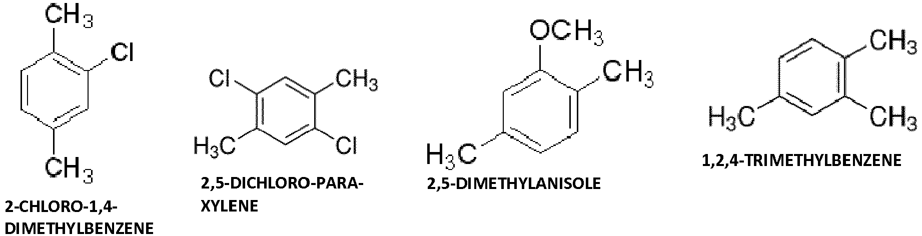

It should be noted that substituents such as the ones noted above (chloro, dichloro, methoxy, and methyl) are not the only aromatic substituents that are capable of being modified by this process into reactive intermediates and their subsequent polymers. Additionally, the paracyclophanes and compounds derived thereof are not exclusive to this process. Meta and ortho orientation of the substituents on the aromatic rings are also viable reaction starting materials. The reaction can be generalized to include all compounds that are capable of reaction with nitrous oxide or its intermediate reaction products and also contain hydrogen atoms stabilized by the presence of an aromatic ring. Typically such hydrogen atoms are located in a position alpha to a phenyl ring (benzylic position). Michael structures removed from the alpha aromatic ring positions are known to give similar reactivity to the hydrogen alpha to the aromatic ring position as is well known to those versed in organic synthesis. However, the reactivity of such hydrogen atoms is not limited to alpha and/or Michael positions from an aromatic ring or the aromatic ring such as benzene. Other aromatic stabilization are known for many different rings, fused rings, and non-ring systems, as known to those versed in the art of organic Chemistry. Such starting materials may preferably have the presence of two hydrogen atoms that are capable of being removed to form partially oxidized starting materials. These preferred materials may optionally have the ability to dimerize, trimerize, oligiomerize, or polymerize. The example used in one aspect of this invention is p-xylene.

Alternatively, a plasma gas can be used with the aforementioned starting materials to form the intermediate oxidized products that may subsequently react to form reaction products that are oxidized forms of the starting materials which may be monomers, dimers, trimers, oligomers, or polymers.

Treating the reactive surfaces that may contact the products of these reactions using plasma cleaning of the surface prior to exposure to the reactive intermediate is well known. However, that process is incidental to this method of forming the chemical compounds necessary for the coating or polymer.

A method for the formation of plasma is well documented and known to those familiar in the art of plasma formation. An example, Reaction 2, of such a plasma reaction utilizing the method similar to described in Reaction 1, is another aspect for this general method.

Since the reaction is similar for all compounds used for this method, the use of p-xylene will be used for discussion purposes. It is clear to those versed in the art of Chemistry that the utilization of similar compounds would give similar results, and therefore exhaustive discussion of structural differences in reactivity would add little, if anything, to the teachings of this discovery.

### Reaction 2:

To a quartz tube of 0.95 cm (3/8") diameter and 30.5 cm (12") long is attached a 0.15 cm (1/16") 316 stainless steel tube connected to a gaseous source (such as argon or nitrogen). The stainless steel tube is positioned such that it is a short distance from a grounded electrode (approximately 5 mm to 15 mm). A plasma generator (InfoUnlimited PVM-400, 20 to 50 kHz, 0 to 6000V) is connected to the 0.15 cm (1/16") tube and the grounded electrode. The grounded electrode is positioned and connected such that the gases after having passed over the grounded electrode are allowed to mix with an argon/p-xylene mixture. The resultant mixture is allowed to pass through a 0.95 cm (3/8") diameter X 30.5 cm (12") tube at a temperature ranging from ambient to 800° C. The electric power is supplied to the generator sufficient to allow reaction of the xylene or other starting materials to proceed mostly to completion. Full reaction of the starting material is not necessary. At lower temperatures (ambient to 500° C), a solid is formed in the exit tube. At high temperatures (500 to 650°C) the output of the tube can be mixed with cooling gases and/or directed to a cooled solid or liquid target to condense dimer. At even higher temperatures (650 to 800°C) the output of the tube can be mixed with cooling gases and/or directed to a cooled solid or liquid target to condense monomer. Subsequent polymerization of the condensed monomer is likely to occur rapidly.

Substitution of nitrogen for argon and/or other essentially inert gases are possible without substantially deviating from this procedure. Additionally, modification of the electrode polarity, electrode materials, containment material, and temperatures are possible without significant deviation from the scope of this invention.

Since condensation of the "monomer" of p-xylene is difficult due to the high temperatures of the reaction, it is advantageous to add cool inert gases to the reaction products. Methods for doing this are very simple and well known.

In place of the cool gas method for cooling the reaction products, there is some advantage to allowing the reaction to proceed at a higher pressure and allowing the reaction products to expand into a lower pressure environment. Joule-Thomson cooling occurs, and the reaction products are very rapidly cooled. Subsequent condensation onto the target can then take place with a lower heat load on the target.

The above reactions can be modified as described below.

### Reaction Modification 1: Control of Permittivity

To the output of the reactions described above, the gaseous stream of reaction product is directed to a cool solid surface. The surface target is immersed in a magnetic field such as that provided by a Neodymium magnet (S84, K&J Magnetics). Condensation of the monomer and subsequent polymerization can proceed rapidly while in the magnetic field. If the target and the magnet maintain the same relative orientation during the polymerization process, then a baseline increase in the electrical permittivity will occur. If the orientation of the magnetic field relationship to the target is rotated during the polymerization or solid phase condensation process, then the resulting permittivity will be lessen.

When the reaction is conducted as noted above, using the p-xylylene monomer as the polymerization molecule, but without the presence of the magnetic field the relative permittivity of the material deposited is approximately 3. When the material is run as described with a magnetic flux density of approximately 0.02 to 0.2 Tesla (200 to 2000 Gauss), the relative permittivity is approximately 7. Thus, the magnetic field substantially increased the permittivity by over a factor of 2 times. In a similar manner other salts, dipoles, and salts of organic acids can be entropically oriented during solidification or polymerizations to produce enhanced high permittivity materials. Improvements in permittivity range from 10 to over 1000%.

### Reaction Modification 2: Control of Electrostatic Packing Orientation

To the output of the reactions described above, the gaseous stream of reaction product is directed to a cool solid surface. The surface target is immersed in an electric field such as that provided by a high voltage power supply (G40, Emco, lead spacing 5.1 cm (2") at 4000V). Condensation of the monomer and subsequent polymerization can proceed rapidly while in the electric field. If the target and the electric field maintain the same relative orientation during the polymerization process, then a baseline increase in the electrical permittivity will occur. If the orientation of the electric field relationship to the target is rotated during the polymerization or solid phase condensation process, then the resulting permittivity will be lessen.

When the reaction is conducted as noted above, using the maleic acid salt with guanidine as the high dielectric material, but without the presence of the electric field the relative permittivity of the material deposited is approximately 500. When the material is run as described with an electric field density of 10,000 to 30,000 V/m, the relative permittivity is approximately 25000 to 40000. Thus, the electric field substantially increased the permittivity by at least a factor of 25 in that particular case. In a similar manner other salts, dipoles, and salts of organic acids can be entropically oriented during solidification or polymerizations to produce enhanced high permittivity materials. Improvements in permittivity range from 50 to over 10000%.

The use of electrical and/or magnetic fields during the condensation process will modify the mechanical strength. The material may not be anisotropic after condensation in strong fields. Thus, this method is a way of controlling the mechanical properties of the reaction products made by this procedure.

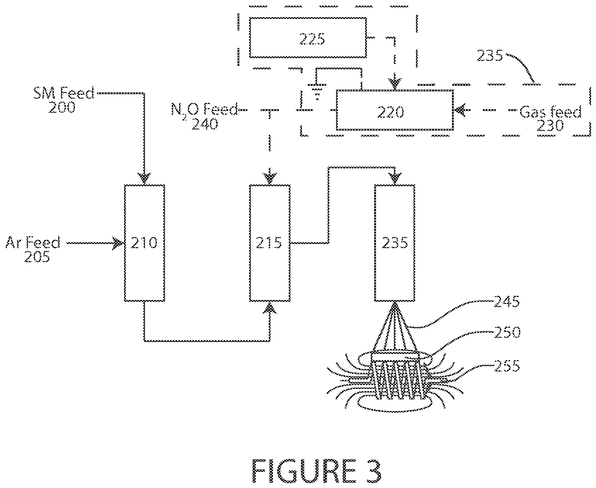

Referring now to Figure 3, a high level flowchart that illustrates an exemplary method of producing an augmented permittivity material according to principles of the present disclosure is shown. Sections, referred to chambers, may comprise tanks having an inlet and an outlet or tubular structures with an inlet and an outlet. Chamber 210 is a heated tube or other evaporation device to volatilize starting material feed 200. Starting material feed 200 is evaporated and mixed with inert gas 205 in chamber 210. Inert gas 205 may be any of a group of inert gases, such as, but not limited to, Argon. Substitution of nitrogen for argon and/or other essentially inert gases is possible. Pumps and valves may be used to propel and control the flow of fluids from one station to another.

By way of example and not limitation, chamber 210 may comprise an electrically heated Inconel (nickel alloy 600) pyrolysis reaction tube. The tube is heated to a temperature of about 450°C to 630°C at atmospheric pressure. A flowing stream of argon gas alone, or with a reactive compound such as nitrous oxide, is supplied to the pyrolysis reaction tube. The starter material feed 200 may be xylene vapor (Aldrich #134449-4L). If the carrier gas 205 includes a reactive compound (e.g., N₂O), the ratio of gases is adjusted to provide approximately molar stoichiometric ratios of 1:1 (xylene to nitrous oxide).

The heated starter material 200 in the volatile mixture with inert gas reacts with monatomic oxygen in reaction chamber 215. Being very reactive and transient, monatomic oxygen must be available to react with the volatile mixture in the reaction chamber 215. As discussed above, the source of monatomic oxygen may be a gaseous compound supplied with the carrier gas 205, or a gaseous compound supplied separately 240, or another source, such as a plasma generator 235.

Monatomic oxygen plasma may be created by exposing oxygen (O₂) gas at a low pressure to a high power energy source, such as an RF discharge, which ionizes the gas. Alternatively, a compound such as Nitrous Oxide (N₂O) may supply monatomic oxygen for the reaction. Thus, a monatomic oxygen plasma generator 235, or a monatomic oxygen chemical compound (e.g., N₂O) feed 240, or another suitable source of monatomic oxygen is provided.

A plasma gas can be used with the aforementioned starting materials to form the intermediate oxidized products that may subsequently react to form reaction products that are oxidized forms of the starting materials which may be monomers, dimers, trimers, oligomers, or polymers. The plasma generator 235 includes a gas feed 230 that supplies gas to a plasma reaction chamber 220. A plasma driver 225 provides high power energy to ionize the gas.

The ratio of gases is adjusted to provide approximately molar stoichiometric ratios of 1:1 (xylene to nitrous oxide or xylene to monatomic oxygen plasma). Illustratively, increased amounts of nitrous oxide result in partial and/or complete oxidation of xylene with reduced formation of the desired cyclophane or its polymer. Close control of the stoichiometry is desired in this gas phase reaction.

The reaction products are supplied to a reaction chamber 235, which is heated to approximately 450° C to 800° C to facilitate vaporization of the reaction products. The vaporized reaction products 245are expelled onto a low temperature collection surface 250, where the reaction products condense and form a solid. At higher temperatures (650° C to 800°C) the output of the reaction chamber 235 is sufficiently hot enough to maintain the monomeric p-xylylene in monomeric form.

Condensation of the gas onto a cooled glass vessel resulted in the deposition of a colorless to cream colored solid. This solid is partially soluble in 95% ethanol. The solid was compared to a sample of [2,2']paracyclophane (Aldrich #P225-5G-A) by GC analysis (SRI#310, 15m, megabore column, FID detector) and was shown to give identical retention times.

Rapidly cooling of the monomer onto a surface 250 results in a liquid condensation of the monomer and rapid polymerization of the monomer into a polymer. Comparison of the film thus produced appears to be identical to parylene film produced by the Gorham process. Without augmentation, permittivity of the solidified product is about 3, electric breakdown strengths are about identical at 100 V/micron, and solubility in both hot and cold solvents are below detectable levels.

In this reaction it is believed that the reactive p-xylylene reactive intermediate is formed and subsequently dimerized in the reaction tube 235 or during condensation 245 onto the substrate 250. This reaction used to synthesize the dimer, in comparison with the known "Gorham process", results in a vast improvement in the overall synthesis yield of the dimer and also results in a vast improvement in the purity of the dimer directly from the reaction. It is understood that variation in the stoichiometric amounts of the reactants may be adjusted to provide for greater or lesser yield with associated purities varying to provide a more economical process or better overall production efficiency without substantially deviating from the scope of the present disclosure. Subsequent purifications of the materials from this reaction can be performed on this material in a manner that is much easier to accomplish than with previously taught processes. The reaction is shown below.

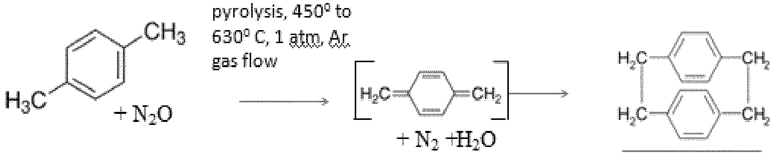

As the reaction temperature at station 235 is increased to >650°C, the deposition of the xylylene monomer can proceed directly onto a solid substrate target without necessity for isolating the intermediate dimer. Deposition of the exit gas at above 650°C reaction temperature upon a cool glass plate resulted in formation of an ethanol insoluble substance that displays characteristics of a parylene polymer. However, solubility characteristics clearly show that the material is insoluble in all common solvents (i.e. hexane, xylene, ethyl acetate, ethanol, water).

It is believed that the reaction mechanism proceeds through a route involving the prior decomposition of nitrous oxide. Nitrous oxide is energetically unstable molecule that can be thermally decomposed at elevated temperatures. Products of the reaction are diatomic nitrogen and monoatomic oxygen. The monoatomic oxygen is able to react with itself to form diatomic oxygen, but this reaction is relatively slow. Estimates vary determining the temperature that pure thermal decomposition occurs, but estimates of 1100° C are often cited. Catalysis of this reaction as shown below in equation 1 is known to occur with a variety of metal oxides and mixed metal oxides. Some temperatures used for nitrous oxide decomposition with certain catalysts are as low as 350° C.

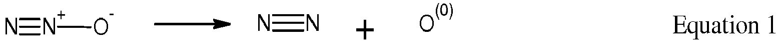

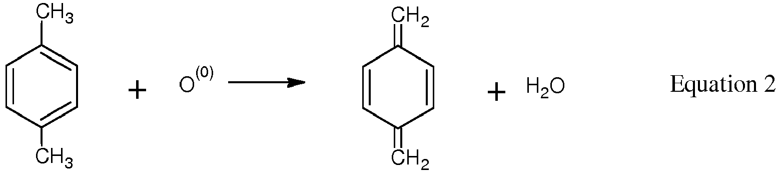

The reactive species for the process is very likely the monoatomic oxygen produced from the decomposition of the nitrous oxide. In this sense, the nitrous oxide can be viewed as a convenient carrier for the delivery of the reactive intermediate monoatomic oxygen.

In a similar manner to the nitrous oxide reaction, pure diatomic oxygen can be utilized as a reactant. However, to produce substantial yields of the desired products, activation of the oxygen is necessary. It is believed that activation of the oxygen is due to the excitation of the oxygen molecule to produce monoatomic oxygen as shown in Equation 3.

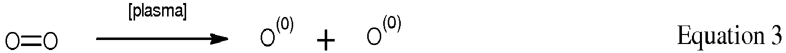

The reaction with monoatomic oxygen produced in this manner thus proceeds in a manner similar to that of the nitrous oxide decomposition route.

Cooling of the elevated temperature gases 245 exiting from the reaction tube 235 is necessary. If the reaction gas is at too high of a temperature, the ability of the reactive intermediate to condense and adhere to a surface is greatly reduced. To this end, a device to mix cool nonreactive gases into the hot reaction stream has been devised. The reaction may proceed at increased pressure (above atmospheric pressure). Accordingly, an expansion valve may be used at the exit of the reaction tube 235 to provide Joule-Thomson effect cooling of the hot gas when the gas is below its inversion temperature.

The method may be extended to other substrates such as the ones shown below.

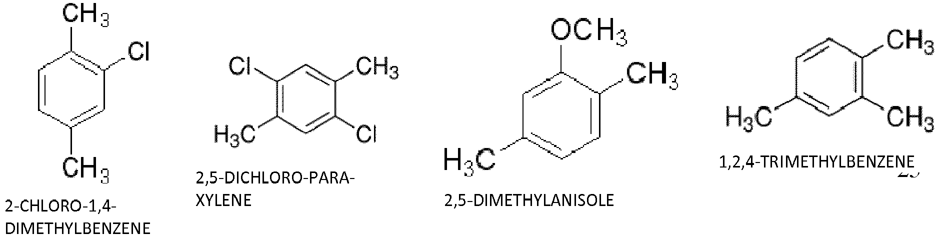

A preferred implementation of the present disclosure augments permittivity of the polymer by exposing the condensing reaction products 245 to a magnetic or electric field. To the output of the reactions described above, the gaseous stream of reaction product 245 is directed to a cool solid surface 250. Illustratively, the surface target 250 may be immersed in a magnetic field 255 such as that provided by a Neodymium magnet (S84, K&J Magnetics). Other magnetic field sources may be utilized and are intended to come within the scope of the present disclosure. Condensation of the monomer and subsequent polymerization can proceed rapidly while in the magnetic field 255. If the target and the magnet maintain the same relative orientation during the polymerization process, then a baseline increase in the electrical permittivity will occur. If the orientation of the magnetic field 255 relationship to the target is rotated during the polymerization or solid phase condensation process, then the resulting permittivity will be decreased.

When the reaction is conducted as noted above, using the p-xylylene monomer as the polymerization molecule, but without the presence of the magnetic field the relative permittivity of the material deposited is approximately 3. When the material is run as described with a magnetic flux 255 density of approximately 0.02 to 0.2 Tesla (200 to 2000 Gauss), the relative permittivity is approximately 7. Thus, the magnetic field substantially increases the permittivity by over a factor of 2 times. In a similar manner other salts, dipoles, and salts of organic acids can be entropically oriented during solidification or polymerizations to produce enhanced high permittivity materials. Improvements in permittivity range from 10 to over 1000% may be attained.

In another implementation, the surface target 250 is immersed in an electric field 255 such as that provided by a high voltage power supply (G40, Emco, lead spacing 5.1 cm (2") at 4000V). Condensation of the monomer and subsequent polymerization can proceed rapidly while in the electric field. If the target and the electric field maintain the same relative orientation during the polymerization process, then a baseline increase in the electrical permittivity will occur. If the orientation of the electric field relationship to the target is rotated during the polymerization or solid phase condensation process, then the resulting permittivity will be lower.

Condensation of dielectric reaction products in the presence of an electric and/or magnetic field, augments the permittivity of the condensed dielectric. This step may be applied to compounds other than parylene polymers.

When the condensation step is conducted as noted above, using maleic acid salt with guanidine as a high dielectric material, but without the presence of the electric field the relative permittivity of the material deposited is approximately 500. When the material is run as described with an electric field density of 10,000 to 30,000 V/m, the relative permittivity is approximately 25000 to 40000. Thus, the electric field substantially increases the permittivity by at least a factor of 25 in that particular case. In a similar manner other salts, dipoles, and salts of organic acids can be entropically oriented during solidification or polymerizations to produce enhanced high permittivity materials. Improvements in permittivity range from 50 to over 10000%.

The use of electrical and/or magnetic fields during the condensation process modifies the mechanical strength. The material may not be anisotropic after condensation in strong fields. Thus, this method is a way of controlling the mechanical properties of the reaction products made by this procedure.

## Claims

1. A method for producing dimers, trimers, oligomers, and polymers from the oxidation of a hydrogen atom alpha to an aromatic ring comprising:
introducing an organic starting material containing at least one hydrogen atom alpha to an aromatic ring into a flowing stream of gas,
mixing said organic starting material in the stream of gas with nitrous oxide;
reacting said hydrogen atom alpha with the aromatic ring to form a reaction product; and
heating said flowing stream of gas with the reaction product to a reaction temperature to allow oxidation of the hydrogen atom alpha and subsequent formation of an output compound from the group consisting of monomers, dimers, trimers, oligomers, and polymers, according to said reaction temperature.

2. A method as in claim 1 wherein the organic starting material comprises xylene.

3. A method as in claim 1 wherein there are two hydrogen atoms alpha to the same aromatic ring.

4. A method as in claim 1 wherein the aromatic ring is benzene.

5. A method as in claim 1 wherein the output compound is mixed with a cool inert gas.

6. A method as in claim 1 wherein the pressure of the flowing stream of gas with the reaction product being heated to a reaction temperature is increased by applying a back pressure device.

7. A method as in claim 1 further comprising a step of mixing a plasma gas with said flowing stream of gas and the organic starting material and nitrous oxide, forming a reaction mixture.

8. A method as in claim 1 further comprising a step of exposing the output compound to a magnetic field during condensation.

9. A method as in claim 1 further comprising a step of exposing the output compound to an electric field during condensation.

## Patentansprüche

1. Verfahren zur Herstellung von Dimeren, Trimeren, Oligomeren und Polymeren durch die Oxidation eines Wasserstoffatoms Alpha an einen aromatischen Ring, umfassend:
Einführen eines organischen Ausgangsmaterials, das mindestens ein Wasserstoffatom Alpha auf einem aromatischen Ring enthält, in einen fließenden Gasstrom,
Mischen des organischen Ausgangsmaterials in dem Gasstrom mit Stickoxid;
Umsetzen des Wasserstoffatoms Alpha mit dem aromatischen Ring, um ein Reaktionsprodukt zu bilden; und
Erhitzen des fließenden Gasstroms mit dem Reaktionsprodukt auf eine Reaktionstemperatur, um Oxidation des Wasserstoffatoms Alpha und nachfolgende Bildung einer Ausgabeverbindung aus der Gruppe bestehend aus Monomeren, Dimeren, Trimeren, Oligomeren und Polymeren gemäß der Reaktionstemperatur zu erlauben.

2. Verfahren nach Anspruch 1, worin das organische Ausgangsmaterial Xylol umfasst.

3. Verfahren nach Anspruch 1, worin es zwei Wasserstoffatome Alpha auf dem gleichen aromatischen Ring gibt.

4. Verfahren nach Anspruch 1, worin der aromatische Ring Benzol ist.

5. Verfahren nach Anspruch 1, worin die Ausgabeverbindung mit einem kühlen Inertgas gemischt wird.

6. Verfahren nach Anspruch 1, worin der Druck des fließenden Gasstroms mit dem auf eine Reaktionstemperatur erhitzten Reaktionsprodukt durch Anwendung einer Gegendruckeinrichtung erhöht wird.

7. Verfahren nach Anspruch 1, ferner umfassend einen Schritt des Mischens eines Plasmagases mit dem fließenden Gasstrom und dem organischen Ausgangsmaterial und Stickoxid, wodurch ein Reaktionsgemisch gebildet wird.

8. Verfahren nach Anspruch 1, ferner umfassend einen Schritt des Aussetzens der Ausgabeverbindung an ein magnetisches Feld während der Kondensation.

9. Verfahren nach Anspruch 1, ferner umfassend einen Schritt des Aussetzens der Ausgabeverbindung an ein elektrisches Feld während der Kondensation.

## Revendications

1. Procédé de production de dimères, de trimères, d'oligomères et de polymères à partir de l'oxydation d'un atome d'hydrogène alpha d'une chaîne aromatique, le procédé consistant à :
introduire un matériau organique de départ contenant au moins un atome d'hydrogène alpha d'une chaîne aromatique dans un flux gazeux en circulation ;
mélanger ledit matériau organique de départ dans le flux gazeux avec de l'oxyde nitreux ;
faire réagir ledit atome d'hydrogène alpha avec la chaîne aromatique pour former un produit de réaction ; et
chauffer ledit flux gazeux en circulation avec le produit de réaction à une température de réaction pour permettre l'oxydation de l'atome d'hydrogène alpha et la formation ultérieure d'un composé de sortie du groupe constitué de monomères, dimères, trimères, oligomères et polymères selon ladite température de réaction.

2. Procédé selon la revendication 1, dans lequel le matériau organique de départ comprend le xylène.

3. Procédé selon la revendication 1, dans lequel il y a deux atomes d'hydrogène alpha dans la même chaîne aromatique.

4. Procédé selon la revendication 1, dans lequel la chaîne aromatique est le benzène.

5. Procédé selon la revendication 1, dans lequel le composé de sortie est mélangé avec un gaz inerte froid.

6. Procédé selon la revendication 1, dans lequel la pression du flux gazeux en circulation avec le produit de réaction chauffé à une température de réaction est augmentée en appliquant un dispositif de contre-pression.

7. Procédé selon la revendication 1, comprenant en outre une étape consistant à mélanger un gaz plasma avec ledit flux gazeux en circulation et le matériau organique de départ et l'oxyde nitreux pour former un mélange de réaction.

8. Procédé selon la revendication 1, comprenant en outre une étape consistant à exposer le composé de sortie à un champ magnétique pendant la condensation.

9. Procédé selon la revendication 1, comprenant en outre une étape consistant à exposer le composé de sortie à un champ électrique pendant la condensation.
